(19) Europäisches Patentamt

European Patent Office

Office européen des brevets

(11) EP 0 573 684 B1

(12) **EUROPÄISCHE PATENTSCHRIFT**

(45) Veröffentlichungstag und Bekanntmachung des Hinweises auf die Patenterteilung:
**17.12.1997 Patentblatt 1997/51**

(51) Int. Cl.$^6$: **A61N 1/365**

(21) Anmeldenummer: **92109688.9**

(22) Anmeldetag: **09.06.1992**

(54) **Verfahren und Vorrichtung zum Ermitteln eines physiologischen Funktionsparameters eines Lebewesens**

Physiological functional parameter sensing method and device of a living creature

Procédé et dispositif de détection d'un paramètre fonctionnel physiologique d'un être vivant

(84) Benannte Vertragsstaaten:
**DE FR GB IT NL SE**

(43) Veröffentlichungstag der Anmeldung:
**15.12.1993 Patentblatt 1993/50**

(73) Patentinhaber: **Pacesetter AB**
**171 95 Solna (SE)**

(72) Erfinder:
- **Franksson, Agneta**
  **S-115 24 Stockholm (SE)**
- **Nilsson, Kenth**
  **S-S-184 60 Akersberga (SE)**

- **Norén, Kjell**
  **S-171 58 Solna (SE)**

(74) Vertreter:
**Lettström, Richard Wilhelm**
**H. Albihns Patentbyra AB,**
**Box 3137**
**103 62 Stockholm (SE)**

(56) Entgegenhaltungen:
| | |
|---|---|
| EP-A- 0 059 868 | EP-A- 0 249 819 |
| EP-A- 0 249 824 | EP-A- 0 249 825 |
| EP-A- 0 331 309 | EP-A- 0 392 800 |
| EP-A- 0 447 024 | US-A- 4 940 053 |
| US-A- 5 025 784 | |

**Beschreibung**

Die Erfindung betrifft ein Verfahren zum Ermitteln eines physiologischen Funktionsparameters eines Lebewesens, wobei mittels einer Meßanordnung ein sich in Abhängigkeit von dem Funktionsparameter änderndes Meßsignal erfaßt wird und wobei Signalanteile eines anderen, das Meßsignal ebenfalls beeinflussenden und dabei eine charakteristische Frequenz aufweisenden Parameters durch Filterung aus dem Meßsignal herausgetrennt werden. Die Erfindung betrifft ferner eine entsprechende Anordnung zum Ermitteln des physiologischen Funktionsparameters.

Ein derartiges, aus der US-A-4 702 253 bekanntes Verfahren dient zur Ermittlung der Atmungsaktivität eines Patienten. Dazu wird mit Hilfe von im Bereich des Herzens des Patienten angeordneten Elektroden und einer an diesen angeschlossenen Meßanordnung die Blutimpedanz gemessen, die sich in Abhängigkeit von der Atmung aber auch in Abhängigkeit von der Herztätigkeit des Patienten ändert. Bei dem bekannten verfahren wird das Meßsignal in einer Abtastanordnung mit einer Abtastfrequenz von 100 Hz abgetastet und dann einer Filteranordnung zugeführt, in der die mit der Atmung korrelierenden niederfrequenten Signalanteile im Bereich von 0,05 Hz bis 1 Hz aus dem Meßsignal herausgefiltert werden. Aus den herausgefilterten niederfrequenten Signalanteilen wird anschließend das Atemminutenvolumen bestimmt und zur Frequenzsteuerung eines Herzschrittmachers herangezogen. Bei dem bekannten Verfahren erfolgt die Herausfilterung der mit der Atmung korrelierenden niederfrequenten Signalanteile mittels eines Bandpassfilters mit festen Grenzfrequenzen. Dabei bleibt jedoch unberücksichtigt, daß je nach körperlicher Belastung oder emotioneller Verfassung des Patienten die Atemfrequenz über einen Frequenzbereich von etwa 0,09 Hz bis 1,1 Hz variieren kann und die Herzfrequenz in einem Bereich von etwa 0,9 Hz bis 2,5 Hz variiert. Beide Frequenzbereiche überlappen sich also, so daß die zur Bestimmung des Atemminutenvolumens herangezogenen Signalanteile nicht nur von der Atmung, sondern insbesondere bei niedrigen Herzschlagfrequenzen auch von der Herztätigkeit abhängen.

Ein derartiges Verfahren und Einrichtung ist auch durch der US 5,025,784 vorbekannt und dient zur Ermittlung der Herzaktivität eines Patienten.

Der Erfindung liegt daher die Aufgabe zugrunde, bei einem sich sowohl in Abhängigkeit von einem zu ermittelnden Funktionsparameter als auch in Abhängigkeit von einem anderen Parameter ändernden Meßsignal die Signalanteile des anderen Parameters aus dem Meßsignal effektiv herauszufiltern, auch wenn die Frequenz des anderen Parameters variiert.

Gemäß der Erfindung wird die Aufgabe dadurch gelöst, daß bei dem Verfahren der eingangs angegebenen Art fortlaufend die zur jeweils aktuellen Frequenz des anderen Parameters umgekehrt proportionale Periodendauer ermittelt wird und daß zur Heraustrennung der auf dem anderen Parameter beruhenden Signalanteile aus dem Meßsignal fortlaufend der Mittelwert des Meßsignals über die Periodendauer des anderen Parameters gebildet wird. Der so gebildete Mittelwert entspricht in seinem zeitlichen Verlauf einer Kerbfilterung Meßsignal wobei der durch die Filterung unterdrückte Frequenzbereich sich laufend an die aktuelle Frequenz des zu unterdrückenden Signalanteils anpaßt. Auf diese Weise können beispielsweise die von dem Wechselstromnetz induzierten 50/60 Hz - Störsignale aus einem Elektrokardiogramm herausgefiltert werden.

Für den Fall, daß der andere Parameter aus einzelnen, sich mit der charakteristischen Frequenz wiederholenden Ereignissen besteht, werden in vorteilhafter Weise die Ereignisse detektiert, wobei die jeweils aktuelle Periodendauer des anderen Parameters aus dem zeitlichen Abstand zwischen den beiden zuletzt detektierten Ereignissen ermittelt wird. Solche Ereignisse sind beispielsweise Herzschläge, Armbewegungen, Drucksignale, die bei einer Impedanzmessung im menschlichen Körper mit dem erfindungsgemäßen Verfahren aus dem Impedanzmeßsignal herausgefiltert werden können, wenn durch die Impedanzmessung die Atemtätigkeit des Patienten gemessen werden soll.

Die Ereignisse können in vorteilhafter Weise mittels einer von der Meßanordnung getrennten Detektoreinrichtung detektiert werden. So können zum Beispiel in Bezug auf die bereits erwähnte Impedanzmessung Herzschläge durch einen Herzschlagdetektor wie z.B. QRS-Detektor oder Armbewegungen durch einen Bewegungssensor detektiert werden. Alternativ dazu können aus dem Meßsignal mit den Ereignissen korrelierende Signalanteile herausgefiltert werden und zur Detektion der Ereignisse ausgewertet werden. Eine solche Filterung kann mit einfachen Mitteln erfolgen, weil nur das Ereignis als solches detektiert werden soll, während die Herausfilterung des mit dem Ereignis verbundenen Signalanteils aus dem Meßsignal durch die Mittelwertbildung entsprechend dem erfindugnsgemäßen Verfahren erfolgt.

Die Mittelwertbildung wird in vorteilhafter Weise dadurch vereinfacht, daß das Meßsignal mit einer vorgegebenen Abtastfrequenz abgetastet wird und daß der Mittelwert aus der Summe der während der für den anderen Parameter ermittelten Periodendauer erfaßten Abtastwerte im Verhältnis zu ihrer Anzahl gebildet wird. Dies umfaßt auch die Möglichkeit, daß die Abtastfrequenz in Abhängigkeit von der ermittelten Periodendauer derart geändert wird, daß die Anzahl der Abtastwerte bezogen auf die Periodendauer konstant bleibt.

Um zu verhindern, daß sich bei einer Änderung des Abstandes zwischen zwei aufeinanderfolgenden Ereignissen die Anzahl der zur Bildung des Abtastmittelwertes herangezogenen Abtastwerte sprunghaft ändert, ist entsprechend einer Weiterbildung des erfindungsgemäßen Verfahrens vorgesehen, daß nach der Detektion eines Ereignisses die Anzahl der zwischen diesem Ereignis und dem vorangegangenen Ereignis erfaßten Abtastwerte neu ermittelt wird und daß für die folgenden Abtastzeitpunkte die Anzahl der jeweils zur Bildung des Mittelwertes herangezogenen Abtastwerte ausgehend von dem vor der Detektion gültigen Wert schrittweise an den neu ermittelten Wert angepaßt wird.

Der für die Bildung der Abtastmittelwerte erforderliche Rechenaufwand läßt sich in vorteilhafter Weise dadurch auf ein Minimum reduzieren, daß zu jedem Abtastzeitpunkt der Zählerstand eines Zählers inkrementiert wird, daß bei dem jeweils ersten Abtastzeitpunkt nach der Detektion eines Ereignisses der Zählerstand in ein Zeigerregister übertragen wird und anschließend der Zählerstand auf Null zurückgesetzt wird, daß zu jedem Zeitpunkt der jeweils aktuelle Abtastwert in ein Schieberegister eingelesen wird, wobei die Speicherinhalte des Schieberegisters um jeweils einen Speicherplatz in Schieberichtung verschoben werden, und daß für jeden Abtastzeitpunkt in einer Recheneinheit der Mittelwert aus der Summe der Speicherinhalte von dem in Schieberichtung gesehen ersten Speicherplatz bis zu dem durch den Inhalt des Zeigerregisters bezeichneten Speicherplatz im Verhältnis zu dem Inhalt des Zeigerregisters ermittelt wird.

Dabei wird nach dem Auftreten eines Ereignisses eine mögliche sprunghafte Änderung der Anzahl der zur Bildung des Mittelwertes herangezogenen Abtastwerte dadurch vermieden, daß bei dem jeweils ersten Abtastzeitpunkt nach der Detektion eines Ereignisses der Zählerstand zwischengespeichert und mit dem Inhalt des Zeigerregisters verglichen wird und daß der Inhalt des Zeigerregisters bei den folgenden Abtastzeitpunkten schrittweise an den zwischengespeicherten Zählerstand angepaßt wird.

Die Anzahl der Speicherplätze des Schieberegisters ist durch das Verhältnis der Abtastfrequenz zu der niedrigsten zu erwartenden Ereignisfrequenz (z.B. Herzschlagfrequenz) bestimmt, so daß auch bei extrem niedrigen Ereignisfrequenzen die Speicherkapazität des Schieberegisters zur Bildung der Abtastwerte ausreicht.

Der die Anordnung betreffende Teil der oben angegebenen Aufgabe wird in vorteilhafter Weise dadurch gelöst, daß bei einer Anordnung zum Ermitteln eines physiologischen Funktionsparameters eines Lebewesens mit einer Meßanordnung im Bereich des Herzens zur Bildung eines sich sowohl Abhängigkeit von der Atmung als auch in Abhängigkeit von der Herztätigkeit ändernden Meßsignals, mit einer an der Meßanordnung angeschlossenen Einrichtung zur Abtastung des von der Meßanordnung kommenden Meßsignals mit einer vorgegebenen Abtastfrequenz und mit einer der Abtasteinrichtung nachgeordneten Filteranordnung zur Heraustrennung der der Atmung entsprechenden niederfrequenten Signalanteile aus dem Meßsignal vorgesehen ist, daß die Anordnung einen Herzschlagdetektor aufweist, daß die Filteranordnung ein Schieberegister enthält, in das die Abtastwerte nacheinander eingelesen werden, daß an dem Schieberegister eine Recheneinheit angeschlossen ist, die einen selektiven Zugriff auf die Speicherinhalte der Speicherplätze des Schieberegisters hat, daß der Herzschlagdetektor mit dem Rücksetzeingang eines Zählers verbunden ist, dessen Zähleingang mit der Abtastfrequenz beaufschlagt ist, daß an dem Ausgang des Zählers ein Zeigerregister angeschlossen ist, das über einen Steuereingang mit dem Herzschlagdetektor verbunden ist und beim Auftreten eines Herzschlages den Zählerstand des Zählers übernimmt, und daß die Recheneinheit mit dem Ausgang des Zeigerregisters verbunden ist und bei jedem Abtastzeitpunkt einen Mittelwert erzeugt, der aus der Summe der Speicherinhalte von dem in Schieberichtung gesehen ersten Speicherplatz des Schieberegisters bis zu dem durch den Inhalt des Zeigerregisters bezeichneten Speicherplatz im Verhältnis zu dem Inhalt des Zeigerregisters gebildet wird.

Die erfindungsgemäße Anordnung ist vorzugsweise Bestandteil eines Herzschrittmachers, in dem die zu jedem Abtastzeitpunkt ermittelten Mittelwerte zur Steuerung der Herzschrittmacherfrequenz herangezogen werden.

Zur Erläuterung der Erfindung wird im folgenden auf die Figuren der Zeichnung Bezug genommen; im einzelnen zeigen:

FIG. 1 ein Blockschaltbild eines frequenzgesteuerten Herzschrittmachers, der eine Meßanordnung zur Bildung eines sich in Abhängigkeit von der Atmung ändernden Meßsignals aufweist, welches sich darüber hinaus auch in Abhängigkeit von der Herztätigkeit ändert,

FIG. 2 ein Blockschaltbild einer Filteranordnung zum Heraustrennen der der Atmung entsprechenden niederfrequenten Signalanteile aus dem Meßsignal,

FIG. 3 ein Zeitdiagramm mit Signalverläufen in der in FIG. 2 dargestellten Filteranordnung und

FIG. 4 ein Diagramm mit Abtastwerten des Meßsignales, die in Abhängigkeit von stimulierten und detektierten Herzschlägen zur Bildung von Abtastmittelwerten am Ausgang der Filteranordnung herangezogen werden.

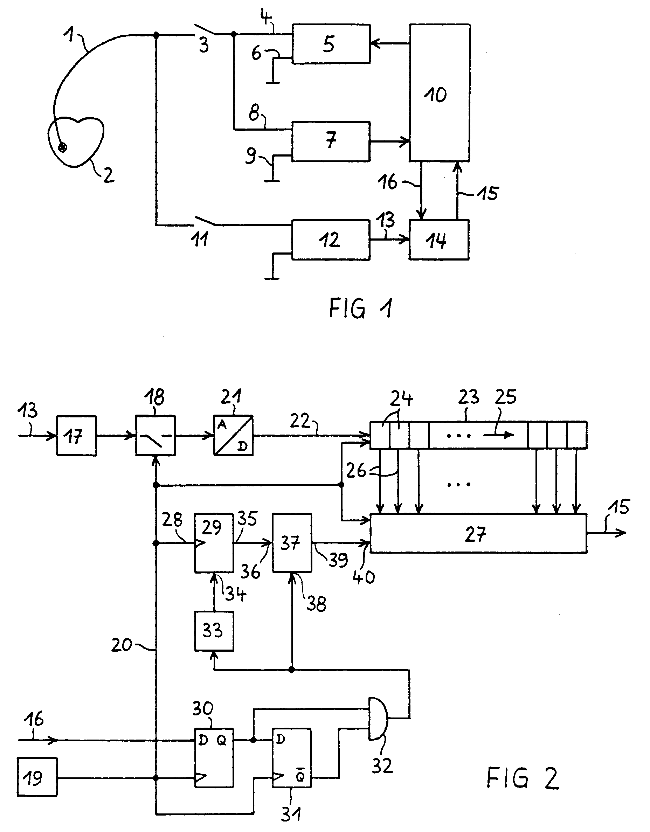

FIG. 1 zeigt das Blockschaltbild eines frequenzgesteuerten Herzschrittmachers, von dem eine Elektrode 1 zu dem Herz 2 eines Patienten führt. Die Elektrode 1 ist über einen steuerbaren Schalter 3 an einem Ausgangsanschluß 4 eines Stimulationsimpulsgenerators 5 angeschlossen, dessen zweiter Ausgangsanschluß 6 mit dem hier nicht gezeigten Gehäuse des Herzschrittmachers verbunden ist. Ein Herzschlagdetektor 7 ist mit einem ersten Eingangsanschluß 8 an dem Ausgangsanschluß 4 des Stimulationsimpulsgenerators 5 angeschlossen und mit seinem zweiten Eingangsanschluß 9 ebenfalls mit dem Herzschrittmachergehäuse verbunden. Der Stimulationsimpulsgenerator 5 und der Herzschlagdetektor 7 sind beide an einer Herzschrittmachersteuerung 10 angeschlossen, die nach jedem stimulierten oder detektierten natürlichen Herzschlag ein Basiszeitintervall startet und die Abgabe eines Stimulationsimpulses durch den Stimulationsimpulsgenerator 5 auslöst, wenn das Basiszeitintervall ausläuft, ohne daß ein natürlicher Herzschlag von dem Herzschlagdetektor 7 detektiert wurde.

An der Elektrode 1 und dem von dem Herzschrittmachergehäuse gebildeten Bezugspotentialanschluß ist ferner über einen weiteren Schalter 11 eine Meßanordnung 12 zur Messung der Impedanz des Körpergewebes zwischen der Elektrode 1 und dem nicht gezeigten Herzschrittmachergehäuse angeschlossen. Die gemessene Impedanz ändert sich dabei sowohl in Abhängigkeit von der Atmung des Patienten als auch in Abhängigkeit von dessen Herztätigkeit. Mit Hilfe der Schalter 3 und 11 lassen sich der Stimulationsimpulsgenerator 5 mit dem Herzschlagdetektor 7 und die Meßanordnung 12 voneinander getrennt an die Elektrode 1 schalten, so daß sie sich nicht gegenseitig beeinflussen können. Das analoge Meßsignal der Meßanordnung 12 wird über eine Ausgangsleitung 13 einer Filteranordnung 14 zugeführt, in der die mit der Atmung korrelierenden niederfrequenten Signalanteile aus dem Meßsignal herausgetrennt und über eine Steuerleitung 15 der Herzschrittmachersteuerung 10 zugeführt werden, in der das Basiszeitintervall in Abhängigkeit von der Atmung des Patienten geändert wird. Bei dem in Figur 1 gezeigten Ausführungsbeispiel erfolgt die Stimulation des Herzens 2, die Detektion von Herzschlägen und die Impedanzmessung jeweils zwischen der Elektrode 1 und dem Herzschrittmachergehäuse. Es ist darüberhinaus natürlich auch möglich, ein mehrpoliges Elektrodensystem vorzusehen und die Stimulation, Detektion und Messung zwischen den einzelnen Elektroden des mehrpoligen Elektrodensystems vorzunehmen. Ferner kann in der Herzschrittmachersteuerung 10 das Basiszeitintervall zusätzlich zu der atmungsabhängigen Steuerung auch in Abhängigkeit von den höherfrequenten, mit der Herztätigkeit korrelierenden Signalanteilen des Meßsignals gesteuert werden. An Stelle der Impedanz können auch andere physiologische Funktionsparameter des Herzens wie z.B. Druck und Durchfluß erfaßt und zur Steuerung des Herzschrittmachers herangezogen werden.

Wie Figur 1 zeigt, ist die Filteranordnung 14 über eine Steuerleitung 16 mit der Herzschrittmachersteuerung 10 verbunden, die auf der Steuerleitung 16 jedesmal dann ein Steuersignal erzeugt, wenn von dem Stimulationsimpulsgenerator 5 ein Herzschlag stimuliert oder von dem Herzschlagdetektor 7 ein natürlicher Herzschlag detektiert worden ist. Es aber auch möglich, die einzelne Herzschlagereignisse aus dem Meßsignal der Meßanordnung 12 durch Hochpassfilterung herauszufiltern.

Ein detailiertes Blockschaltbild der Filteranordnung 14 ist in Figur 2 dargestellt. Das analoge Meßsignal gelangt über die Ausgangsleitung 13 der Meßanordnung 12 auf ein Tiefpass- oder Bandpassfilter 17 und von dort an eine Abtasteinrichtung 18, in der das Meßsignal mit einer von einem Taktgeber 19 erzeugten und über eine Taktsignalleitung 20 der Abtasteinrichtung 18 zugeführten vorgegebenen Abtastfrequenz von beispielsweise 10 Hz abgetastet wird. Die analogen Abtastwerte werden in einem der Abtasteinrichtung 18 nachgeordneten Analog-/Digital-Umsetzer 21 in entsprechende Digitalwerte umgesetzt und über eine Datensignalleitung 22 im Takt der Abtastfrequenz in ein Schieberegister 23 eingelesen, wobei bei jedem erneuten Einlesen eines Abtastwertes die Speicherinhalte der Speicherplätze 24 des Schieberegisters 23 in Richtung des Pfeiles 25 um jeweils einen Speicherplatz 24 verschoben werden. Die Anzahl der Speicherplätze 24 des Schieberegisters 23 ergibt sich aus dem Verhältnis der Abtastfrequenz zu der niedrigsten zu erwartenden Herzschlagfrequenz und beträgt demnach bei einer Abtastfrequenz von 10 Hz und einer minimalen Herzschlagfrequenz von 30 Schlägen je Minute "20". Die einzelnen Speicherplätze 24 sind über Ausgangssignalleitungen 26 mit einer Recheneinheit 27 verbunden, die im Takt der Abtastfrequenz selektiv auf die Speicherinhalte der Speicherplätze 24 zugreift und die so erhaltenen Abtastwerte, wie untenstehend noch näher erläutert wird, zu einem Mittelwert auf der Steuerleitung 15 verknüpft.

Die Taktsignalleitung 20 ist mit dem Zähleingang 28 eines Zählers 29 verbunden, dessen Zählerstand im Takt der Abtastfrequenz, also zu jedem Abtastzeitpunkt inkrementiert wird. Die Steuerleitung 16 ist an dem Triggereingang D und der Taktgeber 19 an dem Takteingang eines D-Flip-Flop 30 angeschlossen, das zusammen mit einem nachgeordneten weiteren D-Flip-Flop 31 und einem mit dem nichtinvertierenden Ausgang Q des ersten D-Flip-Flop 30 und dem invertierenden Ausgang $\overline{Q}$ des zweiten D-Flip-Flop 31 verbundenen UND-Glied 32 ein synchrones Monoflop bildet.

Der Ausgang des UND-Gliedes 32 ist über ein Verzögerungsglied 33 mit einem Rücksetzeingang 34 des Zählers 29 verbunden. Der Ausgang 35 des Zählers 29 ist mit dem Eingang 36 eines Zeigerregisters 37 verbunden, das einen mit dem Ausgang des UND-Gliedes 32 verbundenen Steuereingang 38 aufweist und dessen Ausgang 39 an einem Steuereingang 40 der Recheneinheit 27 angeschlossen ist.

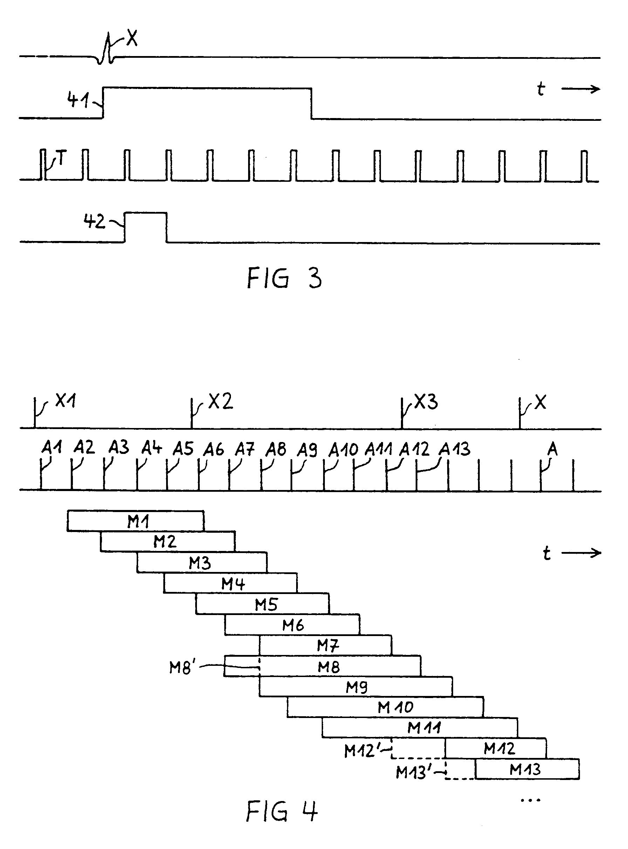

Wie Figur 3 zeigt, wird von der Herzschrittmachersteuerung 10 auf der Steuerleitung 16 nach dem Auftreten eines stimulierten oder detektierten Herzschlages X ein Steuersignal 41 erzeugt, dessen Länge zumindest geringfügig grösser ist, als der durch den Abtastzeittakt T des Taktgebers 19 vorgegebene Abstand zwischen zwei Abtastzeitpunkten. Das synchrone Monoflop 30,31,32 erzeugt an dem Ausgang des UND-Gliedes 32 beim ersten Abtastzeitpunkt nach dem Auftreten des Herzschlages X ein Signal 42, das spätestens mit dem nächstfolgenden Abtastzeitpunkt endet. Das Signal 42 markiert also mit seiner Vorderflanke den jeweils ersten Abtastzeitpunkt nach einem Herzschlag X. Wenn während der Dauer des Steuersignals 41 weitere Herzschläge X auftreten sollten, so wird dies von dem synchronen Monoflop 30,31,32 nicht registriert. Die Dauer des Steuersignals 41 entspricht somit einer Refraktärzeit, innerhalb derer eine Fehldetektion von Herzschlägen für die Filterung des Impedanzsignales vermieden wird. Mit dem Auftreten des Signals 42 wird der Zählerstand des Zählers 29 von dem Zeigerregister 37 übernommen bevor der Zähler 29 nach einer kurzen Verzögerung zurückgesetzt wird. Die Recheneinheit 27 erzeugt bei jedem Abtastzeitpunkt, der ihr über die Taktsignalleitung 20 mitgeteilt wird, einen Mittelwert auf der Steuerleitung 15, der aus der Summe der Speicherinhalte von dem in Schieberichtung 25 gesehen ersten Speicherplatz 24 bis zu dem durch den Inhalt des Zeigerregisters 37 bezeichneten Speicherplatz 24 dividiert durch den Inhalt des Zeigerregisters 37 besteht.

Zur Erläuterung der Funktionsweise der Filteranordnung 14 wird im folgenden auf Figur 4 Bezug genommen, in der in der obersten Zeile das Auftreten von stimulierten oder detektierten Herzschlägen X in Abhängigkeit von der Zeit t dargestellt ist. In der Zeile darunter sind die Abtastwerte A dargestellt, wie sie auf der Datensignalleistung 22 auftreten. Die Abtastwerte A variieren natürlich in Abhängigkeit von der Herztätigkeit und der Atemtätigkeit des Patienten und sind hier nur aus Gründen der Vereinfachung durch jeweils gleichlange Striche markiert. Auch das gezeigte Verhältnis der Abstände zwischen aufeinanderfolgenden Herzschlägen X in Bezug auf den Abstand zwischen zwei aufeinanderfolgenden Abtastzeitpunkten ist hier willkürlich gewählt.

Zum ersten Abtastzeitpunkt A1 nach dem Auftreten des ersten Herzschlages X1 wird der Zählerstand des Zählers 29 auf Null zurückgesetzt und der aktuelle Abtastwert A1 in das Schieberegister 23 eingelesen. Bei jedem weiteren Abtastzeitpunkt wird jeweils ein neuer Abtastwert A2 bis A5 in das Schieberegister 23 eingelesen und jedesmal der Zählerstand inkrementiert. Bei dem ersten Abtastzeitpunkt nach dem nächsten Herzschlag X2 wird der Abtastwert A6 in das Schieberegister 23 eingelesen und der Zähler auf den Zählerstand "5" inkrementiert. Dann wird der Zählerstand "5" in das Zeigerregister 37 übernommen und der Zähler 29 auf Null zurückgesetzt. In der Recheneinheit 27 werden die Speicherinhalte der Speicherplätze 24 angefangen von dem in Schieberichtung 25 gesehen ersten Speicherplatz bis zu dem durch den Inhalt des Zeigerregisters 37 bezeichneten fünften Speicherplatz addiert, so daß die Summe $S = A6 + A5 + A4 + A3 + A2$ erhalten wird. Die Summe S wird durch den Inhalt des Zeigerregisters 37 dividiert, so daß auf der Steuerleitung 15 der Mittelwert $M1 = (A6 + A5 + A4 + A3 + A2) / 5$ erscheint. In Figur 4 ist der Mittelwert M1 durch einen Balken markiert, der die bei der Bildung des Mittelwertes M1 berücksichtigten Abtastwerte A2 bis A6 umfaßt. Beim nächsten Abtastzeitpunkt wird ein neuer Mittelwert M2 aus der Summe des neu hinzugekommenen Abtastwertes A7 und der früheren Abtastwerte A6 bis A3 dividiert durch den Inhalt "5" des Zeigerregisters 37 gebildet. Gleichzeitig wird der Zählerstand des Zählers 29 inkrementiert. Auf gleiche Weise wird für jeden folgenden Abtastzeitpunkt jeweils ein neuer Mittelwert gebildet. Für den letzten Abtastzeitpunkt vor dem dritten Herzschlag X3 ergibt sich dementsprechend ein Mittelwert $M7 = (A12 + A11 + A10 + A9 + A8) / 5$. Der Zählerstand des Zählers 29 beträgt zu diesem Zeitpunkt "6". Beim nächsten Abtastzeitpunkt, der als erster nach dem dritten Herzschlag X3 folgt, wird der Abtastwert A13 in das Schieberegister 23 eingelesen, der Zählerstand des Zählers 29 auf "7" inkrementiert und in das Zeigerregister 37 übertragen und anschließend der Zähler 29 auf Null zurückgesetzt. Als neuer Mittelwert M8 werden die Speicherinhalte des ersten bis zu dem durch den Inhalt des Zeigerregisters 37 bezeichneten siebten Speicherplatz aufsummiert und durch die Zahl 7 in dem Zeigerregister 37 dividiert, so daß gilt: $M8 = (A13 + A12 + A11 + A10 + A9 + A8 + A7) / 7$. Auf entsprechende Weise werden die nächsten Mittelwerte M9 bis M13 und alle weiteren Mittelwerte ermittelt.

Wie Figur 4 bei den Mittelwerten M8 und M12 zeigt, können in Bezug auf die jeweils vorangegangenen Mittelwerte M7 und M11 größere Sprünge in der Anzahl der für die Mittelwertbildung berücksichtigten Abtastwerte eintreten. Diese Sprünge können dadurch vermieden werden, daß die Anzahl der zu berücksichtigenden Abtastwerte schrittweise von dem jeweils früheren Wert an den neuen Wert angepaßt wird, wie dies in der Figur 4 durch die gestrichelten Balken M8' und M9' bzw. M12' und M13' verdeutlicht ist. Dies kann am Beispiel des Blockschaltbildes nach Figur 2 in der Weise erfolgen, daß der von dem Zeigerregister 37 in die Recheneinheit 27 übernommene Zählwert mit einem zuvor in der Recheneinheit 27 zwischengespeicherten Zählwert verglichen wird. Ist der Unterschied kleiner als zwei, so wird der neue Zählwert in der Recheneinheit 27 anstelle des bisherigen Zählwertes zwischengespeichert. Ist dagegen der Unterschied größer oder gleich zwei, so wird der bisher zwischengespeicherte Zählwert in Richtung auf den neuen Zählwert um einen Schritt oder eine vorgegebene Anzahl von Schritten verändert und dann zwischengespeichert. Der jeweils zwischengespeicherte Zählwert wird zur Bestimmung der Anzahl der Abtastwerte bei der Summenbildung und zur nachfolgenden Division verwendet. Auf diese Weise wird bei jedem Abtastzeitpunkt nach dem Auftreten eines Herzschlages die bei der Ermittlung des Mittelwertes zu berücksichtigende Anzahl von Abtastwerten von dem früheren Wert in dem Zeigerregister 37 an den neuen Wert in dem Zeigerregister 37 angepaßt.

Bezugszeichenliste

| | |
|---|---|
| 1 | Elektrode |
| 2 | Herz |
| 3 | steuerbarer Schalter |
| 4 | Ausgangsanschluß von 5 |
| 5 | Stimulationsimpulsgenerator |
| 6 | zweiter Ausgangsanschluß von 5 |
| 7 | Herzschlagdetektor (Ereignisdetektor) |
| 8,9 | Eingangsanschlüsse von 7 |
| 10 | Herzschrittmachersteuerung |
| 11 | weiterer Schalter |
| 12 | Meßanordnung |
| 13 | Ausgangsleitung von 12 |

| 14 | Filteranordnung |
|----|----|
| 15,16 | Steuerleitung |
| 17 | Tiefpaß- oder Bandpaßfilter |
| 18 | Abtasteinrichtung |
| 19 | Taktgeber |
| 20 | Taktsignalleitung |
| 21 | Analog-/Digital- Umsetzer |
| 22 | Datensignalleitung |
| 23 | Schieberegister |
| 24 | Speicherplätze von 23 |
| 25 | Pfeil (=Schieberichtung in 23) |
| 26 | Ausgangssignalleitungen von 23 |
| 27 | Recheneinheit |
| 28 | Zähleingang von 29 |
| 29 | Zähler |
| 30,31 | D-Flip-Flops |
| 32 | UND-Glied |
| 33 | Verzögerungsglied |
| 34 | Rücksetzeingang von 29 |
| 35 | Ausgang von 29 |
| 36 | Eingang von 37 |
| 37 | Zeigerregister |
| 38 | Steuereingang von 37 |
| 39 | Ausgang von 37 |
| 40 | Steuereingang von 27 |
| 41 | Steuersignal |
| 42 | Signal am Ausgang von 32 |
| A,A1-A13 | Abtastwerte |
| M1-M13 | Mittelwerte |
| t | Zeit |
| T | Abtastzeittakt |
| X,X1-X3 | Herzschläge (Ereignisse) |

**Patentansprüche**

1. Verfahren zum Ermitteln eines physiologishen Funktionsparameters eines Lebewesens, wobei mittels einer Meßanordnung (12) ein sich in Abhängigkeit von dem Funktionsparameter änderndes Meßsignal erfaßt wird und wobei Signalanteile eines anderen, das Meßsignal ebenfalls beeinflussenden und eine charakteristische Frequenz aufweisenden Parameters durch Filterung aus dem Meß signal herausgetrennt werden und wobei fortlaufend die zur jeweils aktuellen Frequenz des anderen Parameters umgekehrt proportionale Periodendauer ermittelt wird, dadurch gekennzeichnet daß das Meßsignal mehr als einmal innerhalb einer Periodendauer erfaßt wird und daß zur Heraustrennung der auf dem anderen Parameter beruhenden Signalanteile aus dem Meßsignal fortlaufend zu jedem Erfaßungszeitpunkt der Mittelwert (z.B. M2) des Meßsignals über die Periodendauer des anderen Parameters gebildet wird.

2. Verfahren nach Anspruch 1, **dadurch gekennzeichnet,** daß bei einem, aus einzelnen, sich mit der charakteristischen Frequenz wiederholenden Ereignissen (X) bestehenden anderen Parameter die Ereignisse (X) detektiert werden und daß die jeweils aktuelle Periodendauer des anderen Parameters aus dem zeitlichen Abstand zwischen den beiden zuletzt detektierten Ereignissen (z.B. X2,X1) ermittelt wird.

3. Verfahren nach Anspruch 2, **dadurch gekennzeichnet,** daß die Ereignisse (X) mittels einer von der Meßanordnung (12) getrennten Detektoreinrichtung (7) detektiert werden.

4. Verfahren nach Anspruch 2, **dadurch gekennzeichnet,** daß aus dem Meßsignal mit den Ereignissen (X) korrelierende Signalanteile herausgefiltert werden und zur Detektion der Ereignisse (X) ausgewertet werden.

5. Verfahren nach einem der vorangehenden Ansprüche, **dadurch gekennzeichnet,** daß das Meßsignal mit einer vorgegebenen Abtastfrequenz abgetastet wird und daß der Mittelwert (z.B. M2) aus der Summe der während der für den anderen Parameter ermittelten Periodendauer erfaßten Abtastwerte (A7 bis A3) im Verhältnis zu ihrer Anzahl gebildet wird.

6. Verfahren nach einem der Ansprüche 2 bis 4 und 5, **dadurch gekennzeichnet,** daß nach der Detektion eines Ereignisses (z.B. X3) die Anzahl der zwischen diesem Ereignis (X3) und dem vorangegangenen Ereignis (X2) erfaßten Abtastwerte (A) neu ermittelt wird und daß für die folgenden Abtastzeitpunkte die Anzahl der jeweils zur Bildung des Mittelwertes (M8', M9') herangezogenen Abtastwerte ausgehend von dem vor der Detektion gültigen Wert schrittweise an den neu ermittelten Wert angepaßt wird.

7. Verfahren nach einem der Ansprüche 2 bis 4 und 5, **dadurch gekennzeichnet,** daß zu jedem Abtastzeitpunkt der Zählerstand eines Zählers (29) inkrementiert wird, daß bei dem jeweils ersten Abtastzeitpunkt nach der Detektion eines Ereignisses (X) der Zählerstand in ein Zeigerregister (37) übertragen wird und anschließend der Zählerstand auf Null zurückgesetzt wird, daß zu jedem Abtastzeitpunkt der jeweils aktuelle Abtastwert in ein Schieberegister (23) eingelesen wird, wobei die Speicherinhalte des Schieberegisters (23) um jeweils einen Speicherplatz (24) in Schieberichtung (25) verschoben werden, und daß für jeden Abtastzeitpunkt in einer Recheneinheit (27) der Mittelwert aus der Summe der Speicherinhalte von dem in Schieberichtung (25) gesehen ersten Speicherplatz (24) bis zu dem durch den Inhalt des Zeigerregisters (37) bezeichneten Speicherplatz (24) im Verhältnis zu dem Inhalt des Zeigeregisters (37) ermittelt wird.

8. Verfahren nach Anspruch 3, **dadurch gekennzeichnet,** daß bei dem jeweils ersten Abtastzeitpunkt nach der Detektion eines Ereignisses (X) der Zählerstand zwischengespeichert und mit dem Inhalt des Zeigerregisters (37) verglichen wird und daß der Inhalt des Zeigerregisters (37) bei den folgenden Abtastzeitpunkten schrittweise an den zwischengespeicherten Zählerstand angepaßt wird.

9. Verfahren nach Anspruch 7 oder 8, **dadurch gekennzeichnet,** daß die Anzahl der Speicherplätze (24) des Schieberegisters (23) durch das Verhältnis der Abtastfrequenz zu der niedrigsten zu erwartenden Ereignisfrequenz bestimmt ist.

10. Anordnung zum Ermitteln eines physiologischen Funktionsparameters eines Lebewesens mit einer Meßanordnung (12) im Bereich des Herzens (2) zur Bildung eines sich sowohl in Abhängigkeit von der Atmung als auch in Abhängigkeit von der Herztätigkeit ändernden Meßsignals, mit einer an der Meßanordnung (12) angeschlossenen Abtasteinrichtung (18) zur Abtastung des von der Meßanordnung (12) kommenden Meßsignals mit einer vorgegebenen Abtastfrequenz und mit einer der Abtasteinrichtung (18) nachgeordneten Filteranordnung (14) zur Heraustrennung der der Atmung entsprechenden niederfrequenten Signalanteile aus dem Meßsignal, **dadurch gekennzeichnet,** daß die Anordnung einen Herzschlagdetektor (7) aufweist, daß die Filteranordnung (14) ein Schieberegister (23) enthält, in das die Abtastwerte nacheinander eingelesen werden, daß an dem Schieberegister (23) eine Recheneinheit (27) angeschlossen ist, die einen selektiven Zugriff auf die Speicherinhalte der Speicherplätze (24) des Schieberegisters (23) hat, daß der Herzschlagdetektor (7) mit dem Rücksetzeingang (34) eines Zählers (29) verbunden ist, dessen Zähleingang (28) mit der Abtastfrequenz beaufschlagt ist, daß an dem Ausgang (35) des Zählers (29) ein Zeigerregister (37) angeschlossen ist, das über einen Steuereingang (38) mit dem Herzschlagdetektor (7) verbunden ist und beim Auftreten eines Herzschlages den Zählerstand des Zählers (29) übernimmt und daß die Recheneinheit (27) mit dem Ausgang (29) des Zeigerregisters (37) verbunden ist und bei jedem Abtastzeitpunkt einen Mittelwert erzeugt, der aus der Summe der Speicherinhalte von dem in Schieberichtung (25) gesehen ersten Speicherplatz (24) bis zu dem durch den Inhalt des Zeigerregisters (37) bezeichneten Speicherplatz (24) des Schieberegisters (23) im Verhältnis zu dem Inhalt des Zeigerregisters (37) gebildet wird.

11. Anordnung nach Anspruch 10, **dadurch gekennzeichnet,** daß die Anordnung Bestandteil eines Herzschrittmachers ist, in dem die zu jedem Abtastzeitpunkt ermittelten Mittelwerte zur Steuerung der Herzschrittmacherfrequenz herangezogen werden.

**Claims**

1. Method for establishing a physiological functional parameter of a living being, wherein by means of a measuring arrangement (12), a measured signal which changes as a function of the functional parameter is detected, and wherein signal portions of another parameter, which likewise influences the measured signal and has a characteristic frequency, are separated out from the measured signal by filtering, and wherein the period which is inversely proportional to the frequency of the other parameter that is current in each case is continuously established, characterised in that the measured signal is detected more than once within a period, and in that in order to separate out from the measured signal the signal portions which are based on the other parameter, the average value (e.g. M2) of the measured signal over the period of the other parameter is continuously formed at each detection instant.

2. Method according to claim 1, characterised in that in the case of another parameter which comprises individual events (X) which recur with the characteristic frequency, the events (X) are detected, and in that the period of the other parameter that is current in each case is established from the time interval between the two last detected events (e.g. X2, X1).

3. Method according to claim 2, characterised in that the events (X) are detected by means of a detector device (7) which is separate from the measuring arrangement (12).

4. Method according to claim 2, characterised in that signal portions which correlate with the events (X) are filtered out from the measured signal and evaluated in order to detect the events (X).

5. Method according to one of the preceding claims, characterised in that the measured signal is sampled with a predetermined sampling frequency, and in that the average value (e.g. M2) is formed from the sum of the sampled values (A7 to A3) detected during the period established for the other parameter in relation to the number of said sampled values.

6. Method according to one of the claims 2 to 4 and 5, characterised in that after the detection of an event (e.g. X3), the number of sampled values (A) detected between this event (X3) and the preceding event (X2) is newly established, and in that for the next sampling instants, the number of sampled values used in each case to form the average value (M8', M9'), starting from the value which was valid before the detection, is matched gradually to the newly established value.

7. Method according to one of the claims 2 to 4 and 5, characterised in that at each sampling instant, the counter content of a counter (29) is incremented, in that at the sampling instant which is the first after the detection of an event (X) in each case, the counter content is transferred into a pointer register (37) and the counter content is subsequently reset to zero, in that at each sampling instant, the sampled value which is current in each case is read into a shift register (23), with the memory contents of the shift register (23) being shifted by one memory location (24) in the direction of shifting (25) in each case, and in that for each sampling instant, in an arithmetic unit (27), the average value is established from the sum of the memory contents of the first memory location (24), seen in the direction of shifting (25), up to the memory location (24) which is designated by the content of the pointer register (37), in relation to the content of the pointer register (37).

8. Method according to claim 3, characterised in that at the sampling instant which is the first after the detection of an event (X) in each case, the counter content is stored temporarily and compared with the content of the pointer register (37), and in that the content of the pointer register (37) at the next sampling instants is matched gradually to the temporarily stored counter content.

9. Method according to claim 7 or 8, characterised in that the number of memory locations (24) of the shift register (23) is determined by the ratio of the sampling frequency to the lowest event frequency to be expected.

10. Arrangement for establishing a physiological functional parameter of a living being, having a measuring arrangement (12) in the region of the heart (2) in order to form a measured signal which changes both as a function of the breathing and as a function of the action of the heart, having a sampling device (18) connected to the measuring arrangement (12) for sampling the measured signal coming from the measuring arrangement (12) with a predetermined sampling frequency, and having a filter arrangement (14) arranged downstream of the sampling device (18) for separating out from the measured signal the low-frequency signal portions that correspond to the breathing, characterised in that the arrangement has a heart-beat detector (7), in that the filter arrangement (14) contains a shift register (23), into which the sampled values are read one after the other, in that there is connected to the shift register (23) an arithmetic unit (27) which has a selective access to the memory contents of the memory locations (24) of the shift register (23), in that the heart-beat detector (7) is connected to the reset input (34) of a counter (29), to the counting input (28) of which is applied the sampling frequency, in that there is connected to the output (35) of the counter (29) a pointer register (37), which is connected to the heart-beat detector (7) by way of a control input (38) and takes over the counter content of the counter (29) upon the occurrence of a heart beat, and in that the arithmetic unit (27) is connected to the output (29) of the pointer register (37) and generates at each sampling instant an average value, which is formed from the sum of the memory contents of the first memory location (24), seen in the direction of shifting (25), up to the memory location (24) of the shift register (23) that is designated by the content of the pointer register (37), in relation to the content of the pointer register (37).

11. Arrangement according to claim 10, characterised in that the arrangement is part of a heart pacemaker, in which the average values established at each sampling instant are used to control the heart-pacemaker frequency.

**Revendications**

1. Procédé de détection d'un paramètre fonctionnel physiologique d'un être vivant, un signal de mesure qui varie en fonction du paramètre fonctionnel étant produit au moyen d'un dispositif de mesure (12) et des composantes de signal d'un autre paramètre influençant également le signal de mesure et possédant une fréquence caractéristique étant séparées du signal de mesure par filtrage et la durée de période inversement proportionnelle à la fréquence actuelle de l'autre paramètre étant déterminée en continu, caractérisé en ce que le signal de mesure est détecté plus d'une fois pendant une durée de période et que pour séparer du signal de mesure les composantes de signal de l'autre paramètre, on forme en continu, à chaque moment de détection, la valeur moyenne (par exemple M2) du signal de mesure sur la durée de période de l'autre paramètre.

2. Procédé selon la revendication 1, caractérisé en ce que pour un autre paramètre formé d'événements (X) individuels qui se répètent avec la fréquence caractéristique, on détecte les événements (X) et que la durée de période actuelle de l'autre paramètre est déterminée à partir de l'intervalle de temps entre les deux derniers événements (par exemple X2, X1) détectés.

3. Procédé selon la revendication 2, caractérisé en ce que les événements (X) sont détectés au moyen d'un dispositif de détection (7) séparé du dispositif de mesure (12).

4. Procédé selon la revendication 2, caractérisé en ce qu'on sépare par filtrage du signal de mesure des composantes de signal en corrélation avec les événements (X) et qu'on les exploite pour la détection des événements (X).

5. Procédé selon l'une des revendications précédentes, caractérisé en ce que le signal de mesure est lu avec une fréquence de lecture prédéterminée et que la valeur moyenne (par exemple M2) est formée à partir de la somme des valeurs de lecture (A7 à A3) détectées pendant la durée de période déterminée pour l'autre paramètre, divisée par le nombre de ces valeurs de lecture.

6. Procédé selon l'une des revendications 2 à 4 et 5, caractérisé en ce qu'après la détection d'un événement (par exemple X3), on détermine de nouveau le nombre des valeurs de lecture (A) détectées entre cet événement (X3) et l'événement (X2) précédent et que pour les moments de lecture suivants, on adapte le nombre des valeurs de lecture utilisées pour former la valeur moyenne (M8', M9') progressivement à la nouvelle valeur déterminée, en partant de la valeur valable avant la détection.

7. Procédé selon l'une des revendications 2 à 4 et 5, caractérisé en ce qu'à chaque moment de lecture, on incrémente la position d'un compteur (29), qu'au premier moment de lecture après la détection d'un événement (X), on transmet la position du compteur à un registre (37) pointeur puis on remet le compteur à zéro, qu'à chaque moment de lecture, la valeur de lecture actuelle est entrée dans un registre (23) à décalage, les contenus mémorisés dans le registre (23) à décalage étant décalés chaque fois d'une cellule-mémoire (24) dans le sens (25) de décalage et que pour chaque moment de lecture, on détermine dans une unité (27) de calcul la valeur moyenne en additionnant les contenus des cellules-mémoire, de la première cellule-mémoire (24) vu dans le sens (25) du décalage jusqu'à la cellule-mémoire (24) définie par le contenu du registre (37) pointeur, puis en divisant cette somme par le contenu du registre (37) pointeur.

8. Procédé selon la revendication 3, caractérisé en ce qu'au premier moment de lecture suivant la détection d'un événement (X), on mémorise temporairement la position du compteur et on la compare au contenu du registre (37) pointeur et par le fait qu'aux moments de lecture suivants, on adapte le contenu du registre (37) pointeur par étapes à la position du compteur mémorisée temporairement.

9. Procédé selon la revendication 7 ou 8, caractérisé en ce que le nombre des cellules-mémoire (24) du registre (23) à décalage est déterminé par le rapport de la fréquence de lecture à la fréquence d'événement minimale escomptée.

10. Dispositif de détection d'un paramètre fonctionnel physiologique d'un être vivant comportant un dispositif de mesure (12) dans la région du coeur (2) pour former un signal de mesure qui varie aussi bien en fonction de la respiration qu'en fonction de l'activité cardiaque, comportant un dispositif de lecture (18) raccordé au dispositif de mesure (12) pour lire avec une fréquence de lecture prédéterminée le signal de mesure provenant du dispositif de mesure (12) et comportant un dispositif de filtrage (14) disposé en aval du dispositif de lecture (18) pour séparer par filtrage du signal de mesure les composantes de signal basse fréquence correspondant à la respiration, caractérisé en ce que le dispositif comporte un capteur (7) de battements cardiaques, que le dispositif de filtrage (14) contient un registre (23) à décalage dans lequel les valeurs de lecture sont entrées les unes après les autres, qu'il est raccordé au registre (23) à décalage une unité (27) de calcul qui a un accès sélectif aux contenus des cellules-mémoire (24) du registre (23) à décalage, que le capteur (7) de battements cardiaques est relié à l'entrée (34) de remise à zéro d'un compteur (29) dont l'entrée (28) de comptage est alimentée avec la fréquence de lecture, qu'à la sortie (35) du compteur (29) est raccordé un registre (37) pointeur qui est relié par une entrée (38) de commande au capteur (7) de battements cardiaques et auquel est transmise la position du compteur (29) lors de l'apparition d'un battement cardiaque et que l'unité (27) de calcul est reliée à la sortie (29) du registre (37) pointeur et produit à chaque moment de lecture une valeur moyenne qui résulte de l'addition des contenus des cellules-mémoire (24) du registre (23) à décalage, de la première cellule-mémoire (24), vu dans le sens (25) de décalage, à la cellule-mémoire (24) définie par le contenu du registre (37) pointeur, puis de la division de cette somme par le contenu du registre (37) pointeur.

11. Dispositif selon la revendication 10, caractérisé en ce que le dispositif fait partie d'un stimulateur cardiaque dans lequel les valeurs moyennes déterminées à chaque moment de lecture sont utilisées pour commander la fréquence dudit stimulateur cardiaque.

FIG 1

FIG 2

FIG 3

FIG 4